# EUROPÄISCHE PATENTSCHRIFT

(11) **EP 2 007 459 B1**
(45) Veröffentlichungstag und Bekanntmachung des Hinweises auf die Patenterteilung: **14.09.2016**
(21) Anmeldenummer: 07728097.2
(22) Anmeldetag: 13.04.2007
(51) Int. Cl.: A61M 15/00

(54) **INHALATOR**
INHALER
INHALATEUR

(30) Priorität: 15.04.2006 DE 102006017699
(43) Veröffentlichungstag der Anmeldung: 31.12.2008
(73) Patentinhaber: Boehringer Ingelheim International GmbH, 55216 Ingelheim am Rhein (DE); Boehringer Ingelheim Pharma GmbH & Co. KG, 55216 Ingelheim am Rhein (DE)
(72) Erfinder: WENDLAND, Oliver, 55218 Ingelheim (DE)
(74) Vertreter: Simon, Elke Anna Maria
(86) Internationale Anmeldenummer: PCT/EP2007/053632
(87) Internationale Veröffentlichungsnummer: WO 2007/118858

(56) Entgegenhaltungen:
- EP-A- 0 911 047
- WO-A-94/06498
- WO-A-2006/051300

## Beschreibung

Die Erfindung bezieht sich auf einen Inhalator zur Verabreichung von Arzneimittel in Form von inhalationsfähigen Substanzen, Substanzformulierungen oder -mischungen, insbesondere in Pulverform, mit einem, einen Innenraum zur Aufnahme mindestens einer mit dem Arzneimittel gefüllten Kapsel aufweisenden Gehäuse, das mit einem Mundstück gekoppelt ist, wobei ein Betätiger mit mindestens einer Nadel zum Öffnen der Kapsel gehäuseseitig verschiebbar gelagert ist.

Die EP 0 911 047 A1 offenbart einen Inhalator für die Inhalation pulverförmiger Arzneimittels aus Kapseln, der ein Unterteil mit zwei Fenstern und eine Platte, in der sich Kapsclhalterungen sowie Lufteinlassöffnungen befinden, umfasst. Im Weiteren ist eine Inhalationskammer mit der Platte verbunden, an der ein mit zwei geschliffenen Nadeln versehener, gegen eine Feder beweglicher Betätiger vorgesehen ist. Ein Mundstück ist mit einem Oberteil des Inhalators und ein Deckel klappbar mit dem Unterteil, der Platte sowie dem Oberteil verbunden.

Im Weiteren zeigt die DE 33 45 722 C2 einen Inhalator für die Inhalation pulverförmiger Arzneimittel aus Kapseln, der eine längliche Kammer für die Aufnahme der Kapsel, einen Lufteinlass an dem einen und einen Luftauslass an dem anderen Ende der Kammer aufweist. Darüber hinaus ist eine Öffnungsvorrichtung vorgesehen, mit deren Hilfe die Kapsel seitlich in der Nähe ihrer halbkugelförmigen Enden aufgestochen wird. Die Öffnungsvorrichtung umfasst eine Druckfeder, die eine Schneidvorrichtung in ihrer Normalposition hält.

Bei den bekannten Inhalatoren muss die Kraft der den Betätiger der Schneidvorrichtung zum Öffnen der Kapsel beaufschlagenden Druckfeder derart groß bemessen sein, dass die beispielsweise eine Nadel umfassende.Schneidvorrichtung nach dem Loslassen des Betätigers aus der Kapsel herausbefördert wird, um einen Strömungskanal zum Inhalieren des Arzneimittels freizugeben: Die Druckfederkraft wirkt der Beaufschlagungskraft des Betätigers entgegen, weshalb ein Benutzer des Inhalators eine entsprechend große Druckkraft, die sich im Wesentlichen aus der Druckfederkraft und einer zum Durchdringen der Kapsel mit dem Schneidwerkzeug erforderlichen Öffnungskraft zusammensetzt, aufbringen muss.

Die unten genannten Verbindungen können allein oder in Kombination zur Anwendung in der erfindungsgemäßen Vorrichtung gelangen. In den unten genannten Verbindungen ist W einen pharmakologisch, aktiver Wirkstoff und (beispielsweise) ausgewählt aus der Gruppe bestehend aus Betamimetika, Anticholinergika, Corticosteroiden, PDE4-Inhibitoren, LTD4-Antagonisten, EGFR-Hemmern, Dopamin-Agonisten, HI-Antihistaminika, PAF-Antagonisten und P13-Kinase Inhibitoren. Weiterhin können zwei- oder dreifach Kombinationen von **W** kombiniert werden und zur Anwendung in der erfindungsgemäßen Vorrichtung gelangen. Beispielhaft genannte Kombinationen von **W** wären:
- **W** stellt ein Betamimetika dar, kombiniert mit einem Anticholinergika, Corticosteroide, PDE4-Inhibitore, EGFR-Hemmern oder LTD4-Antagonisten,
- **W** stellt ein Anticholinergika dar, kombiniert mit einem Betamimetika, Corticosteroiden, PDE4-Inhibitoren, EGFR-Hemmern oder LTD4-Antagonisten,
- **W** stellt ein Corticosteroiden dar, kombiniert mit einem PDE4-Inhibitoren, EGFR-Hemmem oder LTD4-Antagonisten
- **W** stellt ein PDE4-Inhibitoren dar, kombiniert mit einem EGFR-Hemmem oder LTD4-Antagonisten
- **W** stellt ein EGFR-Hemmem dar, kombiniert mit einem LTD4-Antagonisten.

Als Betamimetika gelangen hierbei vorzugsweise Verbindungen zur Anwendung, die ausgewählt sind aus der Gruppe bestehend aus Albuterol, Arformoterol, Bambuterol, Bitolterol, Broxaterol, Carbuterol, Clenbuterol, Fenoterol, Formoterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmefamol, Salmeterol, Soterenol, Sulphonterol, Terbutaline, Tiaramide, Tolubuterol, Zinterol, CHF-1035, HOKU-81, KUL-1248 und
- 3-(4-(6-[2-Hydroxy-2-(4-hydroxy-3-hydroxymethyl-phenyl)-ethylamino]-hexyloxy}-butyl)-benzyl-sulfonamid
- 5-[2-(5,6-Diethyl-indan-2-ylamino)-1-hydroxy-ethyl]-8-hydroxy-1H-quinolin-2-on
- 4-Hydroxy-7-[2-{[2-{[3-(2-phenylethoxy)propyl]sulphonyl}ethyl]-amino}ethyl]-2(3H)-benzothiazolon
- 1-(2-Fluor-4-hydroxyphenyl)-2-[4-(1-benzimidazolyl)-2-methyl-2-butylamino]ethanol
- 1-[3-(4-Methoxybenzy]-amino)-4-hydroxyphenyl]-2-[4-(1-benzimidazolyl)-2-methyl-2-butylamino]ethanol
- 1-[2H-5-hydroxy-3-oxo-4H-1,4-benzoxazin-8-yl]-2-[3-(4-N,N-dimethylaminophenyl)-2-methyl-2-propylamino]ethanol
- 1-[2H-5-hydroxy-3-oxo-4H-1,4-benzoxazin-8-yl]-2-[3-(4-methoxyphenyl)-2-methyl-2-propylamino]ethanol
- 1-[2H-5-hydroxy-3-oxo-4H-1,4-benzoxazin-8-yl]-2-[3-(4-n-butyloxyphenyl)-2-methyl-2-propylamino]ethanol
- 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol
- 5-Hydroxy-8-(1-hydroxy-2-isopropylaminobutyl)-2H-1,4-benzoxazin-3-(4H)-on
- 1-(4-Amino-3-chlor-5-trifluormethylphenyl)-2-tert.-butylamino)ethanol
- 6-Hydroxy-8-{1-hydroxy-2-[2-(4-methoxy-phenyl)-1,1-dimethyl-ethylamino]-ethyl}-4H-benzo[1,4]oxazin-3-on
- 6-Hydroxy-8-{1-hydroxy-2-[2-(4-phenoxy-essigsäureethylester)-1,1-dimethyl-ethylamino]-ethyl}-4H-benzo[1,4]oxazin-3-on
- 6-Hydroxy-8-{1-hydroxy-2-[2-(4-phenoxy-essigsäure)-1,1-dimethyl-ethylamino]-ethyl}-4H-benzo[1,4]oxazin-3-on
- 8-{2-[1,1-Dimethyl-2-(2,4,6-trimethylphenyl)-ethylamino]-1-hydroxy-ethyl}-6-hydroxy-4H-benzo[1,4]oxazin-3-on
- 6-Hydroxy-8-{1-hydroxy-2-[2-(4-hydroxy-phenyl)-1,1-dimethyl-ethylamino]-ethyl}-4H-benzo[1,4]oxazin-3-on
- 6-Hydroxy-8-{1-hydroxy-2-[2-(4-isopropyl-phenyl)-1,1dimethyl-ethylamino]-ethyl}-4H-benzo[1,4]oxazin-3-on
- 8-{2-[2-(4-Ethyl-phenyl)-1,1-dimethyl-ethylamino]-1-hydroxy-ethyl}-6-hydroxy-4H-benzo[1,4]oxazin-3-on
- 8-{2-[2-(4-Ethoxy-phenyl)-1,1-dimethyl-ethylamino]-1-hydroxy-ethyl}-6-hydroxy-4H-benzo[1,4]oxazin-3-on
- 4-(4-{2-(2-Hydroxy-2-(6-hydroxy-3-oxo-3,4-dihydro-2H-benzo[1,4]oxazin-8-yl)-ethylamino]-2-methyl-propyl}-phenoxy)-buttersäure
- 8-{2-[2-(3,4-Difluor-phenyl)-1,1-dimethyl-ethylamino]-1-hydroxy-ethyl}-6-hydroxy-4H-benzo[1.4]oxazin-3-on
- 1-(4-Ethoxy-carbonylamino-3-cyano-5-fluorophenyl)-2-(tert.-butylamino)ethanoi
- 2-Hydroxy-5-(1-hydroxy-2-{2-[4-(2-hydroxy-2-phenyl-ethylamino)-phenyl]-ethylamino}-ethyl)-benzaldehyd
- N-[2-Hydroxy-5-(1-hydroxy-2-{2-[4-(2-hydroxy-2-phenyl-ethylamino)-phenyl]-ethylamino}-ethyl)-phenyl]-formamid
- 8-Hydroxy-5-(1-hydroxy-2-{2-[4-(6-methoxy-biphenyl-3-ylamino)-phenyl]-ethylamino)-ethyl)-1H-guinolin-2-on
- 8-Hydroxy-5-[1-hydroxy-2-(6-phenethylamino-hexylamino)-ethyl]-1H-quinolin-2-on
- 5-[2-(2-{4-[4-(2-Amino-2-methyl-propoxy)-phenylamino]-phenyl}-ethylamino)-1-hydroxy-ethyl]-8-hydroxy-1H-quinolin-2-on
- (3-(4-{6-[2-Hydroxy-2-(4-hydroxy-3-hydroxymethyl-phenyl)-ethylamino]-hexyloxy}-butyl)-5-methyl-phenyl]-harnstoff
- 4-(2-{6-[2-(2,6-Dichloro-benzyloxy)-ethoxy]-hexylamino}-1-hydroxy-ethyl)-2-hydroxymethyl-phenol
- 3-(4-{6-[2-Hydroxy-2-(4-hydroxy-3-hydroxymethyl-phenyl)-ethylamino]-hexyloxy}-butyl)-benzylsulfonamid
- 3-(3-{7-[2-Hydroxy-2-(4-hydroxy-3-hydroxymethyl-phenyl)-ethylamino]-heptyloxy}-propyl)-benzylsulfonamid
- 4-(2-{6-[4-(3-Cyclopentanesulfonyl-phenyl)-butoxy]-hexylamino}-1-hydroxy-ethyl)-2-hydroxymethyl-phenol
- N-Adamantan-2-yl-2-(3-{-2-[2-hydroxy-2-(4-hydroxy-3-hydroxymethyl-phenyl)-ethylamino]-propyl}-phenyl)-acetamid

gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Anticholinergika gelangen hierbei vorzugsweise Verbindungen zur Anwendung, die ausgewählt sind aus der Gruppe bestehend aus Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Glycopyrroniumsalzen, bevorzugt das Bromidsalz, Trospiumsalzen, bevorzugt das Chloridsalz, Tolterodin. In den vorstehend genannten Salzen stellen die Kationen die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfönate besonders bevorzugt.

### Ebenfalls bevorzugte Anticholinergika sind ausgewählt aus den Salzen der Formel AC-1

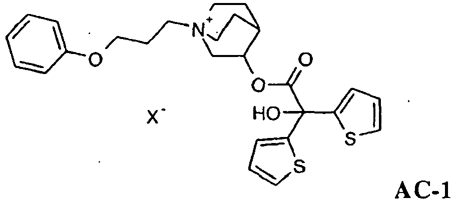

worin X ⁻ ein einfach negativ geladenes Anion, bevorzugt ein Anion ausgewählt aus der Gruppe bestehend aus Fluorid, Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, bevorzugt ein einfach negativ geladenes Anion, besonders bevorzugt ein Anion ausgewählt aus der Gruppe bestehend aus Fluorid, Chlorid, Bromid, Methansulfonat und p-Toluolsulfonat, insbesondere bevorzugt Bromid, bedeutet gegebenenfalls in Form ihrer Racemate, Enantiomere oder Hydrate. Von besonderer Bedeutung sind solche Arcneimittelkombinationen, die die Enantiomere der Formel **AC-1-en** 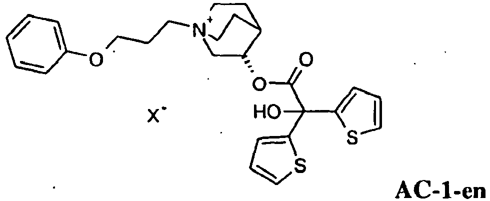 enthalten, worin X ⁻ die vorstehend genannten Bedeutungen aufweisen kann. Weiterhin bevorzugte Anticholinergika sind ausgewählt aus den Salzen der Formel AC-2 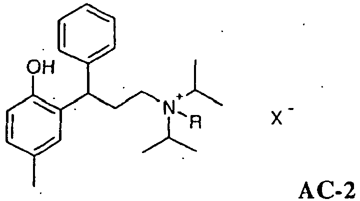 worin R entweder Methyl oder Ethyl bedeuten und worin X ⁻ die vorstehend genanntes Bedeutungen aufweisen kann. In einer alternativen Ausführungsform kann die Verbindung der Formel AC-2 auch in Form der freien Base AC-2-base vorliegen.

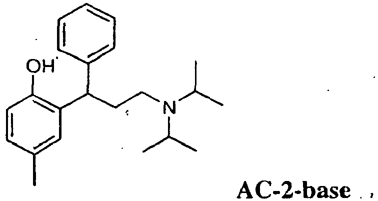

Weiterhin genannte Verbindungen sind:
- 2,2-Diphenylpropionsäuretropenolester-Methobromid
- 2,2-Diphenylpropionsäurescopinester-Methobromid
- 2-Fluor-2.2-Diphenylessigsäurescopinester-Methobromid
- 2-Fluor-2,2-Diphenylessigsäuretropenolester-Methobromid
- 3,3',4,4'-Tetrafluorbenzilsäuretropenolester-Methobromid
- 3,3',4,4'-Tetrafluorbenzilsäurescopinester-Methobromid
- 4,4'-Difluorbenzilsäuretropenolester-Methobromid
- 4,4'-Difluorbenzilsäurescopinester-Methobromid
- 3,3'-Difluorbenzilsäuretropenolester-Methobromid
- 3,3'-Difluorbenzilsäurescopinester-Methobromid
- 9-Hydroxy-fluoren-9-carbonsäuretropenolester-Methobromid
- 9-Fluor-fluoren-9-carbonsäuretropenolester-Methobromid
- 9-Hydroxy-fluoren-9-carbonsäurescopinester-Methobromid
- 9-Fluor-fluoren-9-carbonsäurescopinester-Methobromid
- 9-Methyl-fluoren-9-carbonsäuretropenolester-Methobromid
- 9-Methyl-fluoren-9-carbonsäurescopinester-Methobromid
- Benzi Isäurecyclopropyltropinester-Methobromid
- 2,2-Diphenylpropionsäurecyclopropyltropinester-Methobromid
- 9-Hydroxy-xanthen-9-carbonsäurecyclopropyltropinester-Methobromid
- 9-Methyl-flüoren-9-carbonsäurecyclopropyltropinester-Methobromid
- 9-Methyl-xanthen-9-carbonsäurecyclopropyltropinester-Methobromid
- 9-Hydroxy-fluoren-9-carbonsäurecyclopropyltropinester-Methobromid
- 4,4'-Difluorbenzilsäuremethylestercyclopropyltropinester-Methobromid
- 9-Hydroxy-xanthen-9-carbonsäuretropenolester-Methobromid
- 9-Hydroxy-xanthen-9-carbonsäurescopinester-Methobromid
- 9-Methyl-xanthen-9-carbonsäuretropenolester-Methobromid
- 9-Methyl-xanthen-9-carbonsäurescopinester-Methobromid
- 9-Ethyl-xanthen-9-carbonsäuretropenolester-Methobromid
- 9-Difluormethyl-xanthen-9-carbonsäuretropenolester-Methobromid
- 9-Hydroxymethyl-xanthen-9-carbonsäurescopinester-Methobromid Die vorstehend genannten Verbindungen sind im Rahmen der vorliegenden Erfindung auch als Salze einsetzbar, in denen statt des Methobromids, die Salze Metho-X zur Anwendung gelangen, wobei X die vorstehend für X genannten Bedeutungen haben kann.

Als Corticosteroide gelangen hierbei vorzugsweise Verbindungen zur Anwendung, die ausgewählt sind aus der Gruppe bestehend aus Beclomethason, Betamethason, Budesonid, Butixocort, Ciclesonid, Deflazacort, Dexamethason, Etiprednol, Flunisolid, Fluticason, Loteprednol, Mometason, Prednisolon, Prednison, Rofleponid, Triamcinolon, RPR-106541, NS-126, ST-26 und
- 6,9-Difluor-17-[(2-furanylcarbonyl)oxy]-11-hydroxy-16-methyl-3-oxo-androsta-1,4-dien-17-carbothionsäure (S)-fluoromethylester
- 6,9-Difluor-11-hydroxy-16-methyl-3-oxo-17-propionyloxy-androsta-1,4-dien-17-carbothionsäure (S)-(2-oxo-tetrahydro-furan-3S-yl)ester,
- 6α,9α-difiuoro-11β-hydroxy-16α-methyl-3-oxo-17α-(2,2,3,3-tertamethylcyclo-propylcarbonyl)oxy-androsta-1,4-diene-17β-carbonsäure cyanomethyl ester
gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Dichloroacetate, Propionate, Dihydrogenphosphate, Palmitate, Pivalate oder auch Furoate.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), Tofimilast, Pumafentrin, Lirimilast, Arofyllin, Atizoram, D-4418, Bay-198004, BY343, CP-325,366, D-4396 (Sch-351591), AWD-12-281 (GW-842470), NCS-613, CDP-840, D-4418, PD-168787, T-440, T-2585, V-11294A, Cl-1018, CDC-801, CDC-3052, D-22888, YM-58997, Z-15370 und
- N-(3,5-Dichloro-1-oxo-pyridin-4-yl)-4-difluormethoxy-3-cyclopropylmethoxybenzamid
- (-)p-[(4*a*R*,10*b*S*)-9-Ethoxy-1,2,3,4,4a,10b-hexahydro-8-methoxy-2-methylbenzo[s][1,6]naphthyridin-6-yl]-N,N-diisopropylbenzamid
- (R)-(+)-1-(4-Brombenzyl)-4-[(3-cyclopentyloxy)-4-methoxyphenyl]-2-pyrrolidon
- 3-(Cyclopentyloxy-4-methoxyphenyl)-1-(4-N'-[N-2-cyano-S-methyl-isothioureido]benzyl)-2-pyrrolidon
- cis[4-Cyano-4-(3-cyclopentyloxy-4-methoxyphenyl)cyclohexan-1-carbonsäure]
- 2-carbomethoxy-4-cyano-4-(3-cyclopropylmethoxy-4-difluoromethoxy-phenyl)cyclohexan-1-on
- cis[4-Cyano-4-(3-cyclopropylmethoxy-4-difluormethoxyphenyl)cyclohexan-1-ol]
- (R)-(+)-Ethyl[4-(3-cyclopentyloxy-4-methoxyphenyl)pyrrolidin-2-yliden]acetat
- (S)-(-)-Ethyl[4-(3-cyclopentyloxy-4-methoxyphenyl)pyrrolidin-2-yliden]acetat
- 9-Cyclopentyl-5,6-dihydro-7-ethyl-3-(2-thienyl)-9*H*-pyrazolo[3,4-c]-1,2,4-triazolo[4,3-a]pyridin
- 9-Cyclopentyl-5,6-dihydro-7-ethyl-3-(*tert*-butyl)-9H-pyrazolo[3,4-c]-1,2,4-triazolo[4,3-a]pyridin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der PDE4-Inhibitoren ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTD4-Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321 und
- 1-(((R)-(3-(2-(6,7-Difluor-2-quinolinyl)ethenyl)phenyl)-3-(2-(2-hydroxy-2-propyl)phenyl)thio)methylcyclopropan-essigsäure,
- 1-(((1(R)-3(3-(2-(2,3-Dichlorthieno[3,2-b]pyridin-5-yl)-(E)-ethenyl)pheny)-3-(2-(1-hydroxy-1-methylethyl)phenyl)propyl)thio)methyl)cyclopropanessigsäure
- [2-[[2-(4-tert-Butyl-2-thiazolyl)-5-benzofuranyl]oxymethyl]phenyl]essigsäure gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-Antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als EGFR-Hemmer gelangen hierbei vorzugsweise Verbindungen zur Anwendung, die ausgewählt sind aus der Gruppe bestehend aus Cetuximab, Trastuzumab, ABX-EGF, Mab ICR-62 und
- 4-[(3-Chlor-4-fluorphenyl)amino]-6-{[4-(morpholin-4-yl)-1-oxo-2-buten-1-yl]amino}-7-cyclopropylmethoxy-chinazolin
- 4-[(3-Chlor-4-fluorphenyl)amino]-6-{[4-(N,N-diethylamino)-1-oxo-2-buten-1-yl]amino}-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopropylmethoxy-chinazolin
- 4-[(R)-(1-Phenyl-ethyl)amino]-6-{[4-(morpholin-4-yl)-1-oxo-2-buten-1-yl]amino}-7-cyclopentyloxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin
- 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin
- 4-[(3-Chlor-4-fluor-phenyl)amino]-6-[2-((S)-6-methyl-2-oxo-morpholin-4-yl)-ethoxy]-7-methoxy-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopentyloxy-chinazolin
- 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin
- 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin
- 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin
- 4-[(R)-(1-Phenyl-ethyl)amino]-6-({4-[N-(tetrahydropyran-4-yl)-N,methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin
- 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin
- 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin
- 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin
- 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin
- 4-[(3-Ethinyl-phenyl)amino]-6,7-bis-(2-methoxy-ethoxy)-chinazolin
- 4-[(3-Chlor-4-fluorphenyl)amino]-7-[3-(morpholin-4-yl)-propyloxy]-6-[(vinylcarbonyl)amino]-chinazolin
- 4-[(R)-(1-Phenyl-ethyl)amino]-6-(4-hydroxy-phenyl)-7H-pyrrolo[2,3-d]pyrimidin
- 3-Cyano-4-[(3-chlor-4-fluorphenyl)amino]-6-{[4-(N,N-dimethylamino)-1-oxo-2-buten-1-yl]amino}-7-ethoxy-chinolin
- 4-{[3-Chlor-4-(3-fluor-benzyloxy)-phenyl]amino}-6-(5-{[(2-methansulfonyl-ethyl)amino]methyl}-furan-2-yl)chinazolin
- 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin
- 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin
- 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin
- 4-[(3-Ethinyl-phenyl)amino]-6-{[4-(5,5-dimethyl-2-oxo-morpholin-4-yl)-1-oxo-2-buten-1-yl]amino}-chinazolin
- 4-[(3-Chlor-4-fluor-phenyl)amino]-6-[2-(2,2-dimethyl-6-oxo-morpholin-4-yl)-ethoxy]-7-methoxy-chinazoliri
- 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin
- 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin
- 4-[(3-Chlor-4-fluor-phenyl)amino]-6-{2-[4-(2-oxo-morpholin-4-yl)-piperidin-1-yl]-ethoxy}-7-methoxy-chinazolin
- 4-[(3-Chlor-4-fluor-phenyl)amino]-6-[1-(tert.-butyloxycarbonyl)-piperidin-4-yloxy]-7-methoxy-chinazolin
- 4-[(3-Chlor-4-fluor-phenyl)amino]-6-(trans-4-amino-cyclohexan-1-yloxy)-7-methoxy-chinazolin
- 4-[(3-Chlor-4-fluor-phenyl)amino]-6-(trans-4-methansulfonylamino-cyclohexan-1-yloxy)-7-methoxy-chinazolin
- 4-[(3-Chlor-4-fluor-phenyl)amino]-6-(tetrahydropyran-3-yloxy)-7-methoxy-chinazolin
- 4-[(3-Chlor-4-fluor-phenyl)amino]-6-(1-methyl-piperidin-4-yloxy)-7-methoxy-chinazolin
- 4-[(3-Chlor-4-fluor-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(methoxymethyl)carbonyl]-piperidin-4-yloxy}-7-methoxy-chinazolin
- 4-[(3-Chlor-4-fluor-phenyl)amino]-6-(piperidin-3-yloxy)-7-methoxy-chinazolin
- 4-[(3-Chlor-4-fluor-phenyl)amino]-6-[1-(2-acetylamino-ethyl)-piperidin-4-yloxy]-7-methoxy-chinazolin
- 4-[(3-Chlor-4-fluor-phenyl)amino]-6-(tetrahydropyran-4-yloxy)-7-ethoxy-chinazolin
- 4-[(3-Chlor-4-fluor-phenyl)amino]-6-((S)-tetrahydrofuran-3-yloxy)-7-hydroxy-chinazolin
- 4-[(3-Chlor-4-fluor-phenyl)amino]-6-(tetrahydropyran-4-yloxy)-7-(2-methoxy-ethoxy)-chinazolin
- 4-[(3-Chlor-4-fluor-phenyl)amino]-6-{trans-4-[(dimethylamino)sulfonylamino]-cyclohexan-1-yloxy}-7-methoxy-chinazolin
- 4-[(3-Chlor-4-fluor-phenyl)amino]-6-{trans-4-[(morpholin-4-yl)carbonylamino]-cyclohexan-1-yloxy}-7-methoxy-chinazolin
- 4-[(3-Chlor-4-fluor-phenyl)amino]-6-{trans-4-[(morpholin-4-yl)sulfonylamino]-cyclohexan-1-yloxy}-7-methoxy-chinazolin
- 4-[(3-Chlor-4-fluor-phenyl)amino]-6-(tetrahydropyran-4-yloxy)-7-(2-acetylamino-ethoxy)-chinazolin
- 4-[(3-Chlor-4-fluor-phenyl)amino]-6-(tetrahydropyran-4-yloxy)-7-(2-methansulfonylamino-ethoxy)-chinazolin
- 4-[(3-Chlor-4-fluor-phenyl)amino]-6-{1-[(piperidin-1-yl)carbonyl]-piperidin-4-yloxy}-7-methoxy-chinazolin
- 4-[(3-Chlor-4-fluor-phenyl)amino]-6-(1-aminocarbonylmethyl-piperidin-4-yloxy)-7-methoxy-chinazolin
- 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)sulfonyl]-N-methyl-amino}-cyclohexan-1-yloxy)-7-methoxy-chinazolin
- 4-[(3-Chlor-4-fluor-phenyl)amino]-6-(trans-4-ethansulfonylamino-cyclohexan-1-yloxy)-7-methoxy-chinazolin
- 4-[(3-Chlor-4-fluor-phenyl)amino]-6-(1-methansulfonyl-piperidin-4-yloxy)-7-ethoxy-chinazolin
- 4-[(3-Chlor-4-fluor-phenyl)amino]-6-(1-methansulfonyl-piperidin-4-yloxy)-7-(2-methoxy-ethoxy)-chinazolin
- 4-[(3-Chlor-4-fluor-phenyl)amino]-6-[1-(2-methoxy-acetyl)-piperidin-4-yloxy]-7-(2-methoxy-ethoxy)-chinazolin
- 4-[(3-Chlor-4-fluor-phenyl)amino]-6-(cis-4-acetylamino-cyclohexan-1-yloxy)-7-methoxy-chinazolin
- 4-[(3-Ethinyl-phenyl)amino]-6-[1-(tert.-butyloxycarbonyl)-piperidin-4-yloxy]-7-methoxy-chinazolin
- 4-[(3-Ethinyl-phenyl)amino]-6-(tetrahydropyran-4-yloxy]-7-methoxy-chinazolin
- 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin
- 4-[(3-Chlor-4-fluor-phenyl)amino]-6-{cis-4-[(morpholin-4-yl)carbonylamino]-cyclohexan-1-yloxy}-7-methoxy-chinazolin
- 4-[(3-Chlor-4-fluor-phenyl)amino]-6-{1-[2-(2-oxopyrrolidin-1-yl)ethyl]-piperidin-4-yloxy}-7-methoxy-chinazolin
- 4-[(3-Chlor-4-fluor-phenyl)amino]-6-{1-[(morpholin-4-yl)carbonyl]-piperidin-4-yloxy}-7-(2-methoxy-ethoxy)-chinazolin
- 4-[(3-Ethinyl-phenyl)amino]-6-(1-acetyl-piperidin-4-yloxy)-7-methoxy-chinazolin
- 4-[(3-Ethinyl-phenyl)amino]-6-(1-methyl-piperidin-4-yloxy)-7-methoxy-chinazolin
- 4-[(3-Ethinyl-phenyl)amino]-6-(1-methansulfonyl-piperidin-4-yloxy)-7-methoxy-chinazolin
- 4-[(3-Chlor-4-fluor-phenyl)amino]-6-(1-methyl-piperidin-4-yloxy)-7(2-methoxy-ethoxy)-chinazolin
- 4-[(3-Chlor-4-fluor-phenyl)amino]-6-(1-isopropyloxycarbonyl-piperidin-4-yloxy)-7-methoxy-chinazolin
- 4-[(3-Chlor-4-fluor-phenyl)amino]-6-(cis-4-methylamino-cyclohexan-1-yloxy)-7-methoxy-chinazolin
- 4-[(3-Chlor-4-fluor-phenyl)amino]-6-{cis-4-[N-(2-methoxy-acetyl)-N-methyl-amino]-cyclohexan-1-yloxy}-7-methoxy-chinazolin
- 4-[(3-Ethinyl-phenyl)amino]-6-(piperidin-4-yloxy)-7-methoxy-chinazolin
- 4-[(3-Ethinyl-phenyl)amino]-6-[1-(2-methoxy-acetyl)-piperidin-4-yloxy]-7-methoxy-chinazolin
- 4-[(3-Ethinyl-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(cis-2,6-dimethyl-morpholin-4-yl)carbonyl]-piperidin-4-yloxy}-7-methoxy-chinazolin
- 4-[(3-Chlor-4-fluor-phenyl)amino]-6-{1-[(2-methyl-morpholin-4-yl)carbonyl]-piperidin-4-yloxy}-7-methoxy-chinazolin
- 4-[(3-Chlor-4-fluor-phenyl)amino]-6-{1-[(S,S)-(2-oxa-5-aza-bicyclo[.2.2.1]hept-5-yl)carbonyl]-piperidin-4-yloxy}-7-methoxy-chinazolin
- 4-[(3-Chlor-4-fluor-phenyl)amino]-6-{1-[(N-methyl-N-2-methoxyethyl-amino)carbonyl]-piperidin-4-yloxy}-7-methoxy-chinazolin
- 4-[(3-Chlor-4-fluor-phenyl)amino]-6-(1-ethyl-piperidin-4-yloxy)-7-methoxy-chinazolin
- 4-[(3-Chlor-4-fluor-phenyl)amino]-6-{1-[(2-methoxyethyl)carbonyl]-piperidin-4-yloxy}-7-methoxy-chinazolin
- 4-[(3-Chlor-4-fluor-phenyl)amino]-6-{1-[(3-methoxypropyl-amino)-carbonyl]-piperidin-4-yloxy}-7-methoxy-chinazolin
- 4-[(3-Chlor-4-fluor-phenyl)amino]-6-[cis-4-(N-methansulfonyl-N-methyl-amino)-cyclohexan-1-yloxy]-7-methoxy-chinazolin
- 4-[(3-Chlor-4-fluor-phenyl)amino]-6-[cis-4-(N-acetyl-N-methyl-amino)-cyclohexan-1-yloxyl-7-methoxy-chinazolin
- 4-[(3-Chlor-4-fluor-phenyl)amino]-6-(trans-4-methylamino-cyclohexan-1-yloxy)-7-methoxy-chinazolin
- 4-[(3-Chlor-4-fluor-phenyl)amino]-6-[trans-4-(N-methansulfonyl-N-methyl-amino)-cyclohexan-1-yloxy]-7-methoxy-chinazolin
- 4-[(3-Chlor-4-fluor-phenyl)amino]-6-(trans-4-dimethylamino-cyclohexan-1-yloxy)-7-methoxy-chinazolin
- 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin
- 4-[(3-Chlor-4-fluor-phenyl)amino]-6-(1-methansulfonyl-piperidin-4-yloxy)-7-methoxy-chinazolin
- 4-[(3-Chlor-4-fluor-phenyl)amino]-6-(1-cyano-piperidin-4-yloxy)-7-methoxy-chinazolin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Dopamin-Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsuifonat.

Als H1-Antihistaminika gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als pharmazeutisch wirksame Substanzen, Substanzformulierungen oder Substanzmischungen werden alle inhalierbaren Verbindungen eingesetzt, wie z.B. auch inhalierbare Makromoleküle, wie in EP 1 003 478 offenbart. Vorzugsweise werden Substanzen, Substanzformulierungen oder Substanzmischungen zur Behandlung von Atemwegserkrankungen eingesetzt, die im inhalativen Bereich Verwendung finden.

Weiterhin kann die Verbindung aus der Gruppe der Derivate von Mutterkornalkaloiden, der Triptane, der CGRP-Hemmern, der Phosphodiesteräse-V-Hemmer stammen, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Als Derivate der Mutterkornalkaloide: Dihydroergotamin, Ergotamin.

Es ist Aufgabe der Erfindung, einen Inhalator der eingangs genannten Art zu schaffen, der leicht zu bedienen ist.

Erfindungsgemäß wird die Aufgabe dadurch gelöst, dass das Mundstück derart mit dem Betätiger in Wirkverbindung steht, dass die Nadel des Betätigers in einer Gebrauchslage des Mundstückes nicht zum Einstechen der Kapsel in den Innenraum ragt, wobei mindestens ein, insbesondere bogenförmiger, Ansatz an dem Mundstück angeordnet ist, der den Betätiger beim Verschwenken des Mundstückes beaufschlagt. Der Ansatz ist derart gestaltet, dass er ab einer bestimmten Schwenkstellung des Mundstückes den Betätiger aus seiner eingeschobenen Endlage, in der die Nadel die Kapsel öffnet, in seine ausgeschobene Ausgangslage verlagert und in dieser hält.

Wird das Mundstück in seine Gebrauchslage gebracht, in der es Bestandteil eines Strömungskanals zur Inhalation des Arzneimittels ist, steht es derart mit dem Betätiger in Wirkverbindung, dass dieser in seine unbeaufschlagte Ausgangslage verschoben und hierbei die Nadel oder ein vergleichbares Schneid- bzw. Stechelement zum Öffnen der Kapsel zur Freigabe der Öffnung des Strömungskanals verlagert wird. Der Betätiger kann an dem Gehäuse oder einem weiteren Bauteil des Inhalators, das beispielsweise die Kapsel aufnimmt, verschiebbar gelagert sein. Als ein wesentlicher Vorteil dieser Lösung kann auf die Anordnung einer das Betätigungselement in seine unbeaufschlagte Ausgangslage verschiebende Druckfeder verzichtet oder eine Druckfeder mit einer relativ geringen Federkraft verwendet werden, womit eine Verringerung der Beaufschlagungskraft des Betätigungselementes zum Öffnen der Kapsel gegenüber dem Stand der Technik einhergeht. Demnach ist der Inhalator leicht und zuverlässig zu bedienen.

In Ausgestaltung ist das Mundstück über ein Gelenk verschwenkbar mit dem Gehäuse verbunden und verschiebt beim Anklappen an das Gehäuse den Betätiger zwangsweise in seine Ausgangslage, in der die Nadel die Kapsel zum Inhalieren freigibt. Aufgrund dieser Maßnahmen ist ein mechanisch verhältnismäßig einfacher Aufbau gewährleistet und der Betätiger wird infolge der an dem leicht handhabbaren Mundstück wirkenden Hebelkräfte unter einer geringen Kraftbeaufschlagung mit seiner Nadel aus der Kapsel herausgezogen, wodurch die Funktion des Inhalators sichergestellt ist, da dieser nur mit dem die Gebrauchslage einnehmenden Mundstück bestimmungsgemäß zu verwenden ist.

Damit sich der Ansatz nicht störend auswirkt, ist zweckmäßigerweise der Ansatz an der in Richtung des Betätigers weisenden Unterseite des Mundstückes angeformt und ragt in der Gebrauchslage des Mundstückes in den Innenraum des Gehäuses.

Um ein definiertes Herausschieben des Betätigers aus seiner eingeschobenen Endlage in seine Ausgangslage sicherzustellen, beaufschlagt bevorzugt der Ansatz einen Anschlag des Betätigers, der sich senkrecht zu der Verschieberichtung des Betätigers erstreckt. Damit der Betätiger bei seiner Beaufschlagung durch den Ansatz nicht verkantet, sind vorzugsweise an dem Betätiger zwei zueinander beabstandete Nadeln übereinander angeordnet und zu beiden Seiten der Nadeln ist jeweils ein Anschlag an dem Betätiger vorgesehen, wobei jedem Anschlag ein Ansatz des Mundstückes zugeordnet ist. Zweckmäßigerweise sind die beiden Anschläge Bestandteil eines in den Betätiger eingesetzten Stiftes.

Grundsätzlich sind der Anschlag des Betätigers und/oder der Ansatz des Mundstückes aus einem handelsüblichen Material gefertigt. Um einen Verschleiß der in der Regel aus Kunststoffen gefertigten Bauteile des Inhalators zu minimieren, ist der Anschlag des Betätigers und/oder der Ansatz des Mundstückes aus einem Metall, insbesondere einem Edelstahl, gefertigt.

Für ein einfaches Öffnen des Inhalators, beispielsweise zum Einsetzen oder Entfernen einer Kapsel, sind vorteilhafterweise die Anschläge des Betätigers und/oder die Ansätze des Mundstückes derart ausgebildet, dass bei einer Beaufschlagung des Betätigers in der Gebrauchslage des Mundstückes ein Abklappen des Mundstückes von dem Gehäuse erfolgt.

Alternativ dazu sind vorzugsweise die Anschläge des Betätigers und/oder die Ansätze des Mundstückes derart ausgebildet, dass bei einer Beaufschlagung des Betätigers in der Gebrauchslage des Mundstückes eine Verschiebung des Betätigers verhindert ist. Somit geben beim Inhalieren die an dem Betätiger befestigten Nadeln die entsprechenden Öffnungen der Kapsel frei.

Um ein Einsetzen der Kapsel zu erleichtern und ein nahezu geräuschfreies Funktionieren des Betätigers zu ermöglichen, ist zwischen dem Betätiger und einem in den Innenraum des Gehäuses ragenden Kapselhalter eine Druckfeder angeordnet, die eine der Gewichtskraft des Betätigers entgegenwirkende Federkraft aufweist. Die Druckfeder weist lediglich eine derart bemessene Federkraft auf, dass sie den Betätiger in seiner Ausgangslage hält, dieser also nicht aufgrund seines Eigengewichtes die eingeschobene Endlage einnimmt.

Um ein positionsgenaues Öffnen der Kapsel, ein Verbiegen der Nadeln beim Einstechen der Kapsel und ein Verkanten des Betätigers bei seiner Verschiebung zu verhindern, ist der Kapselhalter mit Führungsansätzen für die Nadeln des Betätigers versehen.

An dem Gehäuse ist eine damit verrastbare Platte zum Verschließen des Innenraums angelenkt, mit der das Mundstück in seiner Gebrauchslage verrastbar und an der der Kapselhalter befestigt ist. Damit die Ansätze in der Gebrauchslage des Mundstücks in das Gehäuse eintauchen können, sind zweckmäßigerweise in der Platte Freimachungen für die Ansätze des Mundstückes eingelassen. Zum Schutz des Mundstückes ist an dem Gehäuse ein damit verrastbarer Deckel schwenkbar angelenkt. Vorzugsweise sind das Mundstück, die Platte und der Deckel gemeinsam an dem Gehäuse gelagert.

Es versteht sich, dass die vorstehend genannten und nachstehend noch zu erläuternden Merkmale nicht nur in der jeweils angegebenen Kombination, sondern auch in anderen Kombinationen verwendbar sind. Der Rahmen der Erfindung ist nur durch die Ansprüche definiert.

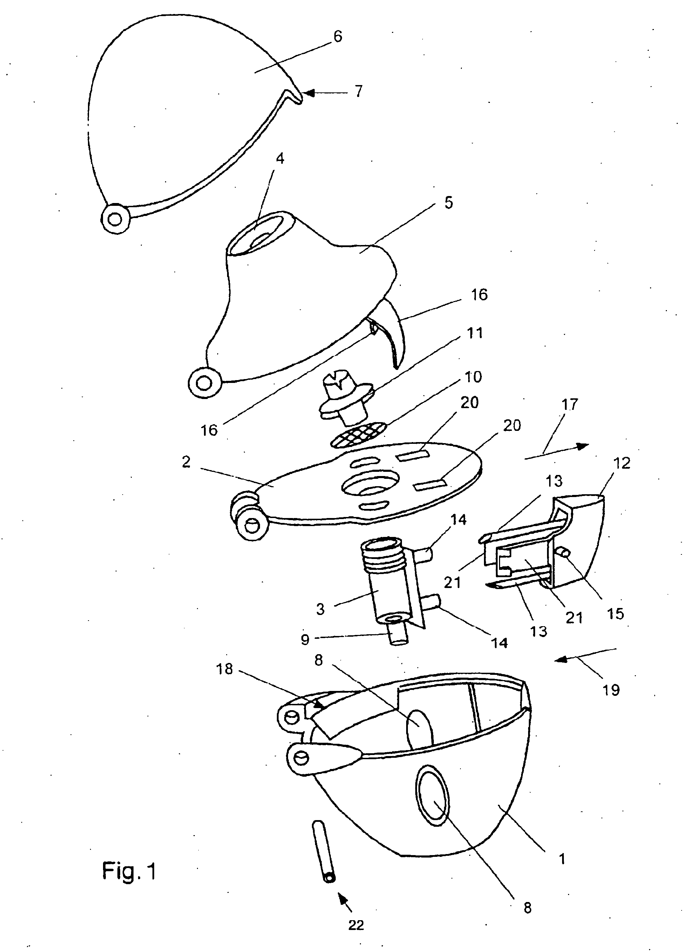

Die Erfindung wird im Folgenden anhand eines Ausführungsbeispieles unter Bezugnahme auf die zugehörige Zeichnung näher erläutert. Die einzige Figur der Anmeldung zeigt eine Explosionsdarstellung eines erfindungsgemäßen Inhalators.

Der Inhalator umfasst ein als Unterteil dienendes Gehäuse 1, an dem eine mit dem Gehäuse 1 verrastbare Platte 2 zum Verschließen des Gehäuses 1 angelenkt ist. Die Platte 2 ist mit einem in dem Gehäuse 1 versenkbaren Kapselhalter 3 zur Aufnahme einer mit einem pulverförmigen Arzneimittel gefüllten Kapsel in einer Kammer versehen und mit einem, einen Inhalationskanal 4 aufweisenden Mundstück 5 in seiner Gebrauchslage verrastbar. Ein Deckel 6, der das in die Gebrauchslage verschwenkte Mundstück 5 in einer Verschlussposition abdeckt, ist mittels eines Verschlusselementes 7 mit dem Gehäuse 1 verrastet. Das Gehäuse 1, die Platte 2, das Mundstück 5 und der Deckel 6 sind durch ein gemeinsames Gelenk (22) verschwenkbar miteinander verbunden. Das Gehäuse 1 ist mit zwei Fenstern 8 im Bereich des Kapselhalters 3 versehen, der an seiner Unterseite eine Lufteinströmöffnung 9 aufweist und an seiner offenen Oberseite mit einem Sieb 10 sowie einem in das Mundstück 5 ragenden Strömungsansatz 11 abgedeckt ist, wobei das Sieb 10 und der Strömungsansatz 11 mit der Platte 2 verbunden sind.

Darüber hinaus ist ein Betätiger 12 in dem Gehäuse 1 verschiebbar gelagert, der zwei in den Kapselhalter 3 einstoßbare Nadeln 13 zum Einstechen der Kapsel umfasst. Die zueinander beabstandeten Nadel 13 sind in Führungsstutzen 14 des Kapselhalters 3 verschiebbar gelagert. Im Weiteren sind an dem Betätiger 12 zwei Führungsansätze 21 zu seiner gehäuseseitigen verschiebbaren und unverlierbaren Lagerung angeformt. Darüber hinaus weist der Betätiger 12 zwei Anschläge 15 auf, die zu beiden Seiten der Nadeln 13 senkrecht zu deren Längserstreckung angeordnet sind und beim Verschwenken des Mundstückes 5 aus der in der Figur dargestellten Ausgangslage in dessen Gebrauchslage von zwei zugeordneten Ansätzen 16, die an der Unterseite des Mundstückes 5 angeordnet sind, beaufschlagt werden, um den Betätiger 12 in Richtung des Pfeils 17 in seine Ausgangslage zu verschieben, in der die Nadeln 13 nicht in die Kapsel ragen.

Im Folgenden werden die wesentlichen Schritte bei der Benutzung des Inhalators erläutert. Zum Einsetzen der Kapsel in den Kapselhalter 3 werden der Deckel 6 und das Mundstück 5 von dem Gehäuse 1 abgeklappt. Nach dem Einsetzen der Kapsel in den Kapselhalter 3 wird der Innenraum 18 des Gehäuses 1 mit der Platte 2 verschlossen und der Betätiger 12 in Richtung des Pfeils 19 beaufschlagt, damit die Nadeln 13 in die Kapsel einstechen und sie damit öffnen kann. Anschließend wird das Mundstück 5 in seine Gebrauchslage verschwenkt, in der es mit der Platte 2 verrastet ist. Beim Verschwenken des Mundstückes 5 tauchen dessen bogenförmige Ansätze 16 durch rechteckförmige Freimachungen 20 in den Innenraum 18 des Gehäuses 1 und kommen an den Anschlägen 15 des Betätigers 12 zur Anlage. Mit der weiteren Schwenkbewegung des Mundstückes 5 geht die zwangsweise Verschiebung des Betätigers 12 in Richtung des Pfeils 17 einher, bis der Betätiger 12 seine Ausgangslage erreicht hat. Da sich in dieser Ausgangslage des Betätigers 12 die Nadeln 13 nicht in der Kapsel befinden, ist ein Strömungskanal zum Inhalieren des Arzneimittels durch einen Be nutzer des Inhalators freigegeben.

## Patentansprüche

1. Inhalator zur Verabreichung von Arzneimittel in Form von inhalationsfähigen Substanzen, Substanzformulierungen oder -mischungen, insbesondere in Pulverform, mit einem einen Innenraum (18) zur Aufnahme mindestens einer mit dem Arzneimittel gefüllten Kapsel aufweisenden Gehäuse (1), das mit einem Mundstück (5) gekoppelt ist, wobei ein Betätiger (12) mit mindestens einer Nadel (13) zum Öffnen der Kapsel gehäuseseitig verschiebbar gelagert ist,
**dadurch gekennzeichnet, dass**
das Mundstück (5) mit dem Betätiger (12) in Wirkverbindung steht in dem mindestens ein Ansatz (16) an dem Mundstück (5) angeordnet ist, der den Betätiger (12) beim Verschwenken des Mundstückes (5) beaufschlagt, so dass die Nadel (13) des Betätigers (12) in einer Gebrauchslage des Mundstückes (5) nicht zum Einstechen der Kapsel in den Innenraum (18) ragt.

2. Inhalator nach Anspruch 1, **dadurch gekennzeichnet, dass** das Mundstück (5) über ein Gelenk (22) verschwenkbar mit dem Gehäuse (1) verbunden ist und beim Anklappen an das Gehäuse (1) den Betätiger (12) zwangsweise in seine Ausgangslage verschiebt, in der die Nadel (13) die Kapsel zum Inhalieren freigibt.

3. Inhalator nach Anspruch 2, **dadurch gekennzeichnet, dass** der Ansatz (16) bogenförmig ist.

4. Inhalator nach einem der Ansprüche 1 bis 3, **dadurch gekennzeichnet, dass** der Ansatz (16) an der in Richtung des Betätigers (12) weisenden Unterseite des Mundstückes (5) angeformt ist und in der Gebrauchslage des Mundstückes (5) in den Innenraum (18) des Gehäuses (1) ragt.

5. Inhalator nach einem der Ansprüche 1 bis 4, **dadurch gekennzeichnet, dass** der Ansatz (16) einen Anschlag (15) des Betätigers (12) beaufschlagt, der sich senkrecht zu der Verschieberichtung (Pfeile 17, 19) des Betätigers (12) erstreckt.

6. Inhalator nach einem der Ansprüche 1 bis 5, **dadurch gekennzeichnet, dass** an dem Betätiger (12) zwei zueinander beabstandete Nadeln (13) übereinander angeordnet sind und zu beiden Seiten der Nadeln (13) jeweils ein Anschlag (15) an dem Betätiger (12) vorgesehen ist, wobei jedem Anschlag (15) ein Ansatz (16) des Mundstückes (5) zugeordnet ist.

7. Inhalator nach Anspruch 6, **dadurch gekennzeichnet, dass** die beiden Anschläge (15) Bestandteil eines in den Betätiger (12) eingesetzten Stiftes sind.

8. Inhalator nach einem der Ansprüche 5 bis 7, **dadurch gekennzeichnet, dass** der Anschlag (15) des Betätigers (12) und/oder der Ansatz (16) des Mundstückes (5) aus einem Metall, insbesondere einem Edelstahl, gefertigt ist.

9. Inhalator nach einem der Ansprüche 6 bis 8, **dadurch gekennzeichnet, dass**.die Anschläge (15) des Betätigers (12) und/oder die Ansätze (16) des Mundstückes (5) derart ausgebildet sind, dass bei einer Beaufschlagung des Betätigers (12) in der Gebrauchslage des Mundstückes (5) ein Abklappen des Mundstückes (5) von dem Gehäuse (1) erfolgt.

10. Inhalator nach einem der Ansprüche 6 bis 8, **dadurch gekennzeichnet, dass** die Anschläge (15) des Betätigers (12) und/oder die Ansätze (16) des Mundstückes (5) derart ausgebildet sind, dass bei einer Beaufschlagung des Betätigers (12) in der Gebrauchslage des Mundstückes (5) eine Verschiebung des Betätigers (12) verhindert ist.

11. Inhalator nach einem der Ansprüche 1 bis 10, **dadurch gekennzeichnet, dass** zwischen dem Betätiger (12) und einem in den Innenraum (18) des Gehäuses (1) ragenden Kapselhalter (3) eine Druckfeder angeordnet ist, die eine der Gewichtskraft des Betätigers (12) entgegenwirkende Federkraft aufweist.

12. Inhalator nach einem der Ansprüche 6 bis 11, **dadurch gekennzeichnet, dass** der Kapselhalter (3) mit Führungsansätzen (14) für die Nadeln (13) des Betätigers (12) versehen ist.

13. Inhalator nach einem der Ansprüche 1 bis 12, **dadurch gekennzeichnet, dass** an dem Gehäuse (1) eine damit verrastbare Platte (2) zum Verschließen des Innenraums (18) des Gehäuses (1) angelenkt ist, mit der das Mundstück (5) in seiner Gebrauchslage verrastbar und an der der Kapselhalter (3) befestigt ist.

14. Inhalator nach Anspruch 13, **dadurch gekennzeichnet, dass** in der Platte (2) Freimachungen (20) für die Ansätze (16) des Mundstückes (5) eingelassen sind.

15. Inhalator nach einem der Ansprüche 1 bis 14, **dadurch gekennzeichnet, dass** an dem Gehäuse (1) ein damit verrastbarer Deckel (6) schwenkbar angelenkt ist.

## Claims

1. Inhaler for administering medicaments in the form of inhalable substances, substance formulations or substance mixtures, especially in the form of a powder, having a housing (1) comprising an interior (18) for accommodating at least one capsule filled with the medicament, said housing being coupled to a mouthpiece (5), while an actuator (12) encompassing at least one needle (13) is mounted so as to be movable towards the housing in order to open the capsule, **characterised in that** the mouthpiece (5) is operatively connected to the actuator (12) by at least one attachment (16) being arranged on the mouthpiece (5) and acting upon the actuator (12) when the mouthpiece (5) is pivoted, such that the needle (13) of the actuator (12) does not project into the interior (18) to pierce the capsule in a position of use of the mouthpiece (5).

2. Inhaler according to Claim 1, **characterised in that** the mouthpiece (5) is pivotably connected to the housing (1) via a joint (22) and when flipped onto the housing (1) it automatically pushes the actuator (12) into its starting position in which the needle (13) exposes the capsule for inhalation.

3. Inhaler according to Claim 2, **characterised in that** the attachment (16) is arcuate.

4. Inhaler according to one of Claims 1 to 3, **characterised in that** the attachment (16) is formed on the underside of the mouthpiece (5), facing in the direction of the actuator (12), and in the position of use of the mouthpiece (5) it projects into the interior (18) of the housing (1).

5. Inhaler according to one of Claims 1 to 4, **characterised in that** the attachment (16) impacts a stop (15) on the actuator (12), which extends perpendicularly to the direction of movement (arrows 17, 19) of the actuator (12).

6. Inhaler according to one of Claims 1 to 5, **characterised in that** two spaced-apart needles (13) are arranged one above the other on the actuator (12) and a stop (15) is provided on the actuator (12) on both sides of the needles (13), each stop (15) being associated with an attachment (16) on the mouthpiece (5).

7. Inhaler according to Claim 6, **characterised in that** the two stops (15) are part of a pin that is inserted in the actuator (12).

8. Inhaler according to one of Claims 5 to 7, **characterised in that** the stop (15) of the actuator (12) and/or the attachment (16) on the mouthpiece (5) is made from a metal, particularly stainless steel.

9. Inhaler according to one of Claims 6 to 8, **characterised in that** the stops (15) of the actuator (12) and/or the attachments (16) on the mouthpiece (5) are constructed such that when the actuator (12) is impacted in the position of use of the mouthpiece (5) the mouthpiece (5) is flipped away from the housing (1).

10. Inhaler according to one of Claims 6 to 8, **characterised in that** the stops (15) of the actuator (12) and/or the attachments (16) on the mouthpiece (5) are constructed such that when the actuator (12) is impacted in the position of use of the mouthpiece (5) the actuator (12) is prevented from moving.

11. Inhaler according to one of Claims 1 to 10, **characterised in that** between the actuator (12) and a capsule holder (3) projecting into the interior (18) of the housing (1) a compression spring is arranged which has a spring force that counteracts the gravitational force of the actuator (12).

12. Inhaler according to one of Claims 6 to 11, **characterised in that** the capsule holder (3) is provided with guide attachments (14) for the needles (13) of the actuator (12).

13. Inhaler according to one of Claims 1 to 12, **characterised in that** jointed to the housing (1) is a plate (2) that can be latched thereto for closing off the interior (18) of the housing (1) and with which the mouthpiece (5) can be latched in its position of use and to which the capsule holder (3) is attached.

14. Inhaler according to Claim 13, **characterised in that** in the plate (2) there are openings (20) for the attachments (16) of the mouthpiece (5).

15. Inhaler according to one of Claims 1 to 14, **characterised in that**, pivotably jointed to the housing (1) there is a cover (6) that can be latched thereto.

## Revendications

1. Inhalateur pour l'administration d'un médicament sous la forme de substances, de formulations ou de mélanges de substances pouvant être inhalés, en particulier sous forme pulvérulente, avec un boîtier (1) présentant un espace intérieur (18) pour recevoir au moins une capsule remplie du médicament, qui est couplé à un embout buccal (5), dans lequel un actionneur (12) avec au moins une aiguille (13) est placé de façon à pouvoir coulisser pour ouvrir la capsule du côté du boîtier,
**caractérisé en ce que**
l'embout buccal (5) est en liaison fonctionnelle avec l'actionneur (12), dans lequel au moins un épaulement (16) est disposé sur l'embout buccal (5) que l'actionneur (12) sollicite en faisant pivoter l'embout buccal (5), de sorte que l'aiguille (13) de l'actionneur (12) ne dépasse pas dans un état d'utilisation de l'embout buccal (5) pour perforer la capsule dans l'espace intérieur (18).

2. Inhalateur selon la revendication 1, **caractérisé en ce que** l'embout buccal (5) est relié au boîtier (1) de façon à pouvoir pivoter par une articulation (22) et lors du rabattement sur le boîtier (1) pousse l'actionneur (12) obligatoirement dans sa position de sortie, dans laquelle l'aiguille (13) libère la capsule pour l'inhalation.

3. Inhalateur selon la revendication 2, **caractérisé en ce que** l'épaulement (16) est en forme d'arc.

4. Inhalateur selon l'une des revendications 1 à 3, **caractérisé en ce que** l'épaulement (16) est formé sur le côté inférieur orienté dans la direction de l'actionneur (12) de l'embout buccal (5) et dépasse à l'état d'utilisation de l'embout buccal (5) dans l'espace intérieur (18) du boîtier (1).

5. Inhalateur selon l'une des revendications 1 à 4, **caractérisé en ce que** l'épaulement (16) sollicite une butée (15) de l'actionneur (12) qui s'étend perpendiculairement à la direction de déplacement (flèches 17, 19) de l'actionneur (12).

6. Inhalateur selon l'une des revendications 1 à 5, **caractérisé en ce que** sur l'actionneur (12), deux aiguilles à distance l'une de l'autre (13) sont disposées l'une au-dessus de l'autre et des deux côtés de l'aiguille (13) respectivement une butée (15) est prévue sur l'actionneur (12), dans lequel un épaulement (16) de l'embout buccal (5) est affecté à chaque butée (15).

7. Inhalateur selon la revendication 6, **caractérisé en ce que** les deux butées (15) sont des composants d'une broche insérée dans l'actionneur (12).

8. Inhalateur selon l'une des revendications 5 à 7, **caractérisé en ce que** la butée (15) de l'actionneur (12) et/ou l'épaulement (16) de l'embout buccal (5) est en métal, en particulier un métal noble.

9. Inhalateur selon l'une des revendications 6 à 8, **caractérisé en ce que** les butées (15) de l'actionneur (12) et/ou les épaulements (16) de l'embout buccal (5) sont conçus de telle sorte que lors d'une sollicitation de l'actionneur (12) à l'état d'utilisation de l'embout buccal (5), un rabattement de l'embout buccal (5) s'effectue du boîtier (1).

10. Inhalateur selon l'une des revendications 6 à 8, **caractérisé en ce que** les butées (15) de l'actionneur (12) et/ou les butées (16) de l'embout buccal (5) sont conçues de telle sorte que lors d'une sollicitation de l'actionneur (12) à l'état d'actionnement de l'embout buccal (5), un déplacement de l'actionneur (12) est empêché.

11. Inhalateur selon l'une des revendications 1 à 10, **caractérisé en ce qu'**entre l'actionneur (12) et un support de capsule (3) dépassant dans l'espace intérieur (18) du boîtier (1) est disposé un ressort de compression qui présente une force de ressort s'opposant à la pesanteur de l'actionneur (12).

12. Inhalateur selon l'une des revendications 6 à 11, **caractérisé en ce que** le support de capsule (3) est doté d'épaulements de guidage (14) pour les aiguilles (13) de l'actionneur (12).

13. Inhalateur selon l'une des revendications 1 à 12, **caractérisé en ce que** sur le boîtier (1) est articulée une plaque (2) pouvant être encliquetée avec celui-ci pour fermer l'espace intérieur (18) du boîtier (1), avec laquelle l'embout buccal (5) peut être encliqueté dans son état d'utilisation et sur laquelle le support de capsule (3) est fixé.

14. Inhalateur selon la revendication 13, **caractérisé en ce que** des évidements (20) pour les épaulements (16) de l'embout buccal (5) sont ménagés dans la plaque (2).

15. Inhalateur selon l'une des revendications 1 à 14, **caractérisé en ce que** un couvercle (6) est articulé de manière pivotante sur le boîtier (1), le couvercle (6) pouvant être encliqueté avec le boîtier (1).
